# EUROPEAN PATENT SPECIFICATION

(11) **EP 3 846 737 B1**
(45) Date of publication and mention of the grant of the patent: **09.11.2022**
(21) Application number: 19778423.4
(22) Date of filing: 06.09.2019
(51) Int. Cl.: A61F 2/00, A61M 25/02, A61B 8/08, G01N 11/04, G01F 1/66, A61B 5/00, A61B 5/20, A61M 25/00, A61B 8/00, G01F 15/075

(54) **LIQUID-VOLUME MEASURING DEVICE**
FLÜSSIGKEITSVOLUMENMESSVORRICHTUNG
DISPOSITIF DE MESURE DE VOLUME DE LIQUIDE

(30) Priority: 07.09.2018 DK PA201870578
(43) Date of publication of application: 14.07.2021
(73) Proprietor: Coloplast A/S, 3050 Humlebaek (DK)
(72) Inventor: JENSEN, Henrik, 2400 Copenhagen NV (DK); BERTELSEN, Kasper, 2300 Copenhagen S (DK)
(86) International application number: PCT/DK2019/050264
(87) International publication number: WO 2020/048576

(56) References cited:
- WO-A1-2019/118929
- JP-A- 2006 317 224
- US-A1- 2011 046 514
- US-A1- 2016 216 141
- US-A1- 2018 136 026
- Transonic: "For Use With TS410 Flow Modules Precision Tubing Flowsensors ME-PXL Clamp-on Flowsensors @BULLET Non-contact Clamp-on Sensors do not break circuit sterility", , 25 February 2013 (2013-02-25), XP055644804, www.transonic.com Retrieved from the Internet: URL:http://zh-hant.transonicasia.com/custo m/assets/Files/Tubing%20Flowsensors%20(RL- 28-fly-A4).pdf [retrieved on 2019-11-20]
- Levitronix: "Ultrasonic Technology Clamp-On Flowmeter D-Series for Flexible Tubing LFSC-D Clamp-On Flowmeters Ultraclean Non-Invasive Flow Measurement", , 15 September 2015 (2015-09-15), XP055644822, Retrieved from the Internet: URL:https://www.levitronix.com/en/leviflow -lfsc-d-series.html?file=files/dl__documen ts/Product%20Brochures/Brochure%20LEVIFLOW %20Clamp-On%20Flex%20Tubing%20English.pdf [retrieved on 2019-11-20]

## Description

### FIELD OF THE INVENTION

The present invention relates to a liquid-volume measuring device configured for measuring the volume of a liquid running through a urinary catheter.

### BACKGROUND OF THE INVENTION

In some urinary conditions, patients are required to use urinary catheters to be able to empty their bladder and/or equivalent. Sometimes, catheter users need to gather information relating to their urinary ouput. Such information may include the volume and/or color of the urinary output, as well as the frequency in which they need to use the urinary catheter.

For example, in some urinary conditions, the patients may not be able to feel when their bladder is full and when they have to empty it. Thus, especially in the beginning of their course of disease, such catheter users need to learn how often they need to empty their bladder, depending on their liquid input, the size of their bladder, etc. However, such urine output can be troublesome to obtain for users. At present, urine measuring devices often include a measuring cup of some kind, into which the user needs to empty the content of the urinary catheter, take read offs, and write down the information. Having to handle both the urinary catheter, the measuring device, a pen and a block of paper, as well as carry these objects around is very unhandy for users. Furthermore, the measuring device will have to be thoroughly washed afterwards.

Hence, an improved measuring device would be advantageous, and in particular a more handy measuring device, allowing the user to easily obtain the urinary output without having to carry around to many objects.

Healthcare professionals, such as medical doctors, may use such information to provide users with the best possible treatment and guidance. However, the users' urinary output is often kept confidential. Therefore, medical companies and hospitals do not have much user data relating to urinary output for different user groups.

Further, it would be advantegous if such urinary output data could be collected from users, such that the users could obtain better guidance from doctors and such that medical companies could improve their products to different types of users with specific needs, if given access to urinary output data from the individual users or aggregated users using the invention.

JP2006317224A discloses a medical tube for measuring flow rate. The flow rate is measured by letting urine pass through a disposable measuring tube formed integrally with a urine bag.

US 2016/216141 A1 discloses a fluid flow meter for estimating the velocity of water or another fluid flowing through a pipe.

### OBJECT OF THE INVENTION

An object of the present invention is to provide an alternative to the prior art. In particular, it may be seen as a further object of the present invention to provide a urine measuring device that solves the above mentioned problems of the prior art.

### SUMMARY OF THE INVENTION

Thus, the above described object and several other objects are intended to be obtained in a first aspect of the invention by providing a hand-held liquid-volume measuring device configured for being clamped onto part of an intermittent urinary catheter and for measuring the volume of a liquid running through the urinary catheter, the hand-held volume measuring device having a housing comprising a first portion and second portion being pivotably connected to each other, the first portion and the second portion each comprising a contact surface having an indentation running through the portion being shaped and dimensioned for partly receing an intermittent urinary catheter, the indentation, in a closed configured of the device, having an inner diameter between 2.7 and 6 mm, the housing further comprising
- liquid-volume measuring means configured for measuring the volume of liquid running through the urinary catheter over a period of time;
- user interaction means for activating and/or deactivating the liquid measuring means;
- a wireless transmission means, which allows data from the liquid-volume measuring means to be transmitted to a personal terminal, such as a computer, a tablet, or a smartphone; and
- an energy source, such as a rechargeable battery.

In some embodiments, the liquid-volume measuring means comprises
- an ultrasound emitting unit, configured for emitting at least one beam or pulse of ultrasound through part of a urinary catheter
- an ultrasound receiving unit, configured for receiving the at least one beam or pulse of ultrasound when it has been emitted through the urinary catheter.

In some embodiments, the device further comprises a processing unit, configured for receiving data from the liquid-volume measuring means and processing the data to obtain processed data including the volume of liquid running through the urinary catheter over a period of time.

In some embodiments, the wireless transmission means allows data from the liquid-volume measuring means and/or the processed data to be transmitted to a terminal, such as a computer, tablet, or smartphone.

In some embodiments, the housing further comprises a display unit, configured to display processed data from the processing unit, including the volume of a liquid running through the urinary catheter over a period of time.

In some embodiments, the housing has a longitudinal shape and less than 15 cm in all dimensions, such as less than 10 cm in all dimensions.

In some embodiments, the housing comprises a first portion and a second portion being pivotably connected to each other and each comprising a contact surface having an indentation running though the portion, shaped and dimensioned for partly receiving a urinary catheter.

Usually, the size (diameter, circumference) of urinary catheters, in particular intermittent urinary catheters, are from size 8 FR to size 18 FR. FR (or French size or Charriere (Ch)) is a standard gauge for urinary catheters approximately corresponding to the outer circumference in mm. More accurately, the outer diameter of the urinary catheter in mm corresponds to FR divided by 3. Thus 8 FR corresponds to a urinary catheter with an outer diameter of 2.7 mm and 18 FR corresponds to a urinary catheter with an outer diameter of 6 mm.

Usually, the length of urinary catheters, in particular intermittent urinary catheters, are adapted to the intended gender, such that male urinary catheters are typically at least 35 cm, such as between 35 cm and 50 cm, whereas female urinary catheters are typically between 70 mm and 150 mm.

In some embodiments, the device has an open configuration and a closed configuration, wherein
- in the open configuration, the first portion and the second portion are only in contact with each other through a pivotable section,
- in the closed configuration, the contact surface of the first portion and the second portion are in contact with each other such that the indentations running through the first and the second portion together enclose the channel which is adapted to be fitted around part of an outer surface of a urinary catheter.

In some embodiments, the device further comprises a locking mechanism, configured such that in the closed configuration, the first portion and the second portion are interlocked by means of the locking mechanism.

In a second aspect, the present invention relates to a system comprising
- the handheld volume measuring device
- a smartphone, tablet, or computer having an application software installed, programmed to receive and process data from the handheld volume measuring device,
wherein the application software is configured, based on input information and/or machine learning, to inform a user of the system of several statistic data, such as through a graphical user interface of the smartphone, tablet, or computer having the application software installed.

The statistic data may include how many times a day the user urinates, the average volume of urine each time they urinate, and the largest volume urinated so far, etc.

Preferably, a user can interact with the application software through a smartphone, tablet, or computer, and submit further data in the application software.

The application software may be programmed such that each user has a profile where they have to input basic information before they can use the application, such as name, country, gender, etc., as well as store data on body type + urinary catheter user + which brand + illness / type of condition.

The application may further be programmed such that data on intake (fluids + solids + medicine) can be input by the user. Such information could also be received from integrations with third party apps, e.g. fitness trackers (work-outs, sweat, etc).

Data received from the liquid-volume measuring means, as well as manually inputs by the user can be stored on the device (urine, condition of urine, leakage, etc.). As the volume and condition of the urine is a function of intake (solids, liquids, medicine) as well as physical exercise, which in turn means that if the user inputs that data in combination with the output, the application software will be able to process all data on one user and provide detailed overview on the individual user as well as aggregated data across all users using the invention.

The application software may be able to process data from the liquid-volume measuring device of the present invention, calculate urine output and store it. The application software may further be programmed to provide statistics, such as tables, graphs, and other forms of reporting on when, where, how much input/output the user has taken in/generated.

The application software may be programmed to learn cause/effect and help the user maintain an overview of timings of urinations, and predict best urination times based on intake, when and where, through machine learning.

The software application may be such that the user can extract data and customize reports which can be shared with doctors and other third parties. Furthermore, the application may be able to store aggregated data across all users and may be able to generate and share customized reports for medical research purposes.

The application software may further be programmed to send information from all users to a server or a cloud, such that all information is collected and can be used for further statistics. Such information could be used by hospitals or medical companies, e.g. to improve guidance from doctors and improve products for different types of users with specific needs.

The first and second aspect of the present invention may each be combined with any of the other aspects. These and other aspects of the invention will be apparent from and elucidated with reference to the embodiments described hereinafter.

### BRIEF DESCRIPTION OF THE FIGURES

The hand-held measuring device according to the present invention will now be described in more detail with regard to the accompanying figures. The figures show one way of implementing the present invention and is not to be construed as being limiting to other possible embodiments falling within the scope of the attached claim set.

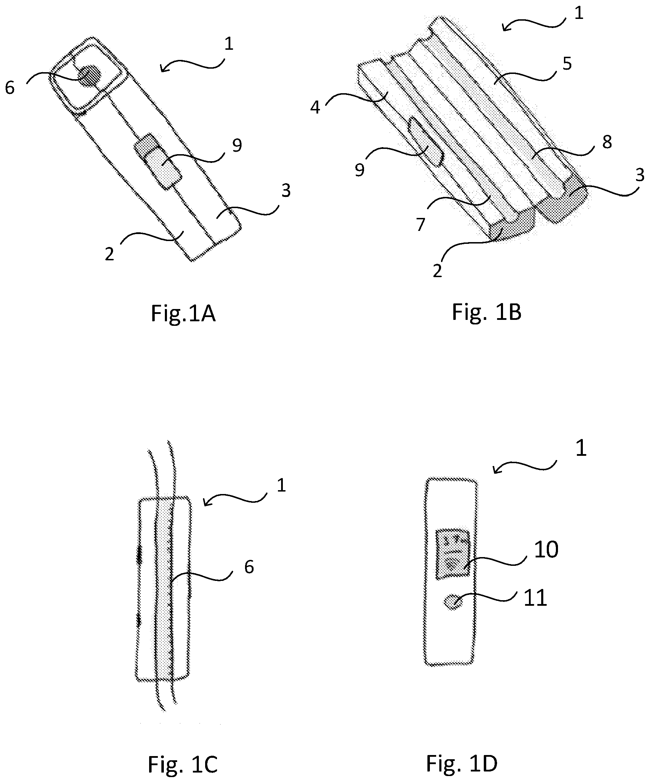

Figures 1A-1D illustrate an embodiment of a hand-held volume measuring device according to an embodiment of the present invention.

### DETAILED DESCRIPTION OF AN EMBODIMENT

Reference is made to Fig. 1A illustrating an embodiment of a hand-held liquid-volume measuring device according to the present invention. The hand-held liquid-volume measuring device (also referred to as "the device") of the present invention is configured for being clamped onto part of a urinary catheter and for measuring the volume of a liquid running through the urinary catheter over a period of time. The hand-held volume measuring device has a housing 1 comprising a channel 6 running through the housing 1. The channel 6 is shaped and dimensioned to be fitted around part of an outer surface of a urinary catheter. The size of the channel 6 may vary, depending on the urinary catheter used by the specific user.

As illustrated in Fig. 1A, the housing 1 may have a longitudinal shape. Preferably, the device is less than 15 cm in all dimensions, such as less than 10 cm in all dimensions. In that way, the device is small enough to be easily transportated in a bag or a pocket, and small enough to be handled with one hand by the user. However, the device cannot be too small, as it has to contain different elements such as an energy source sizable enough to provide long term use. Preferably, the device is made from a material such as plastic, which allows the device to be dropped without breaking. Furthermore, the device has to be water resistant.

As illustrated in Fig. 1B, the housing 1 may comprise a first portion 2 and a second portion 3 being pivotably connected to each other, such as by a hinge of some kind. Both the first portion 2 and the second portion 3 comprise a contact surface 4, 5 having an indentation 7, 8 running through the portion 2, 3, shaped and dimensioned for partly receiving a urinary catheter. Thus, the indentation can, in a closed configuration of the device, have an inner diameter between 2.7 and 6 mm, reflecting common sizes of (intermittent) urinary catheters.

The device has an open configuration and a closed configuration. In the open configuration, which is illustrated in Fig. 1B, the first portion 2 and the second portion 3 are only in contact with each other through a pivotable section, such as a hinge. In the closed configuration, as illustrated in Figs. 1A, 1C, and 1D, the contact surface of the first portion 4 and the contact surface of the second portion 5 are in contact with each other such that the indentations 7, 8 running through the first and the second portion together enclose the channel 6 which is adapted to be fitted around part of an outer surface of a urinary catheter. In that way, when the device is in the open configuration, the user can arrange a urinary catheter within an indentation of either the first portion or the second portion and close the device, by pivoting the contact surface of the first portion and the second portion towards each other. Fig. 1C illustrates a see-through view of a device according to an embodiment of the present invention, where part of a urinary catheter is arranged within the channel 6. Preferably, the device is configured such that it allows the user to secure the device in the closed configuration with one hand and to open the device with one hand. Thereby, the user would be able to measure the volume of urine running through a urinary catheter by clamping the device onto the urinary catheter before urinating and thereafter use the normal procedure to empty the bladder, using only one hand for holding the urinary catheter comprising the device. Furthermore, the device will not be in direct contact with urine, as it is clamped upon the outer surface of the urinary catheter. Thus, the user would in many instances not need to wash the device after use.

As illustrated in Figs. 1A and 1B, the device may further comprise a locking mechanism 9, configured such that in the closed configuration, the first portion 2 and the second portion 3 are interlocked by means of the locking mechanism 9. Preferably, the locking mechanism 9 is a simple locking mechanism allowing the user to secure the device in the closed configuration with one hand and to open the device with one hand.

Furthermore, the hand-held liquid-volume measuring device comprises a liquid-volume measuring means configured for measuring the volume of liquid running through the urinary catheter over a period of time (not illustrated). Preferably, the liquid-volume measuring means uses ultrasound to measure the volume of urine running through the urinary catheter over a period of time and based on a series of measurements throughout the urinating period to measure time of flight and thus the flow of urine. Thus, the liquid-volume measuring means may comprise an ultrasound emitting unit, configured for emitting at least one beam or pulse of ultrasound through part of a urinary catheter and an ultrasound receiving unit, configured for receiving the at least one beam or pulse of ultrasound when it has been emitted through the urinary catheter. The volume of liquid running through the urinary catheter can then be calculated based on data from the liquid-volume measuring means, as well as the diameter of the urinary catheter and the density of the urine running through the urinary catheter. In some embodiments, the liquid measuring device is a ultrasonic flow meter.

Preferably, the device comprises wireless transmission means (not illustrated) allowing data from the liquid-volume measuring means to be transmitted to a terminal, such as a computer, tablet, or smartphone, which provides the processing power needed to calculate the volume of liquid running through the urinary catheter. The wireless transmission means may for example be wifi, Bluetooth, or infared connection means.

In some embodiments, the device itself comprises a processing unit (not illustrated), configured for receiving data from the liquid-volume measuring means and processing the data to obtain processed data including the volume of liquid running through the urinary catheter over a period of time.

If the device comprises a processing unit, the processed data may also be transmitted to a personal terminal, such as a computer, a tablet, or a smartphone via the wireless transmission means. The data from the liquid-volume measuring means and/or the processed data may also be transmitted or uploaded to a server or a cloud.

As illustrated in Fig. 1D, the device may further comprise an user interaction means 11 and a display unit 10.

The display unit 10 may be configured to display processed data, including the volume of a liquid running through the urinary catheter over a period of time.

The user interaction means 11 may be configured for activating and/or deactivating the liquid measuring means. The interaction means may for example be a button which activates the device, including the liquid measuring means when pressed and deactivates the liquid measuring means when released.

The device further comprises an energy source (not illustrated), such as a rechargeable battery, which provides power to the device, such as to the liquid-volume measuring means and the wireless transmitter.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention.

### LIST OF REFERENCE SYMBOLS USED

- 1: housing
- 2: first portion of housing
- 3: second portion of housing
- 4: contact surface of first portion
- 5: contact surface of second portion
- 6: channel
- 7: indentation in first portion
- 8: indentation in second portion
- 9: locking mechanism
- 10: display unit
- 11: user interacting means

## Claims

1. A hand-held liquid-volume measuring device configured for being clamped onto part of an intermittent urinary catheter and for measuring the volume of a liquid running through the urinary catheter, the hand-held volume measuring device having a housing (1) comprising a first portion (2) and a second portion (3) being pivotably connected to each other, the first portion (2) and the second portion (3) each comprising a contact surface (4, 5) having an indentation (7, 8) running through the portion (4, 5) being shaped and dimensioned for partly receiving an intermittent urinary catheter, the indentation, in a closed configuration of the device forming a channel (6) having an inner diameter between 2.7 and 6 mm, the housing (1) further comprising
- liquid-volume measuring means configured for measuring the volume of liquid running through the urinary catheter over a period of time;
- user interaction means (11) for activating and/or deactivating the liquid measuring means;
- a wireless transmission means, which allows data from the liquid-volume measuring means to be transmitted to a personal terminal, such as a computer, a tablet, or a smartphone; and
- an energy source.

2. A hand-held volume measuring device according to claim 1, wherein the liquid-volume measuring means comprises
- an ultrasound emitting unit, configured for emitting at least one beam or pulse of ultrasound through part of a urinary catheter
- an ultrasound receiving unit, configured for receiving the at least one beam or pulse of ultrasound when it has been emitted through the urinary catheter.

3. A hand-held volume measuring device according to any of claims 1-2, wherein the device further comprises a processing unit, configured for receiving data from the liquid-volume measuring means and processing the data to obtain processed data including the volume of liquid running through the urinary catheter over a period of time.

4. A hand-held volume measuring device according to any of claims 1-3, wherein the wireless transmission means allows data from the liquid-volume measuring means and/or the processed data to be transmitted to a terminal, such as a computer, tablet, or smartphone.

5. A hand-held volume measuring device according to any of claims 1-4, wherein the housing further comprises a display unit (10), configured to display processed data from the processing unit, including the volume of a liquid running through the urinary catheter over a period of time.

6. A hand-held volume measuring device according to any of claims 1-5, wherein the housing (1) has a longitudinal shape and less than 15 cm in all dimensions, such as less than 10 cm in all dimensions.

7. A hand-held volume measuring device according to any of claims 1-6, wherein the device has an open configuration and a closed configuration, wherein
- in the open configuration, the first portion (2) and the second portion (3) are only in contact with each other through a pivotable section,
- in the closed configuration, the contact surface of the first portion (4) and the second portion (5) are in contact with each other such that the indentations (7, 8) running through the first portion (2) and the second portion (3) together enclose a channel (6) which is adapted to be fitted around part of an outer surface of a urinary catheter.

8. A hand-held volume measuring device according to any of claims 1-7, wherein the device further comprises a locking mechanism (9), configured such that, in the closed configuration, the first portion (2) and the second portion (3) are interlocked by means of the locking mechanism (9).

## Patentansprüche

1. Handgeführte Flüssigkeitsvolumenmessvorrichtung, die ausgestaltet ist, um auf einen Teil eines intermittierenden Harnkatheters geklemmt zu werden und um das Volumen einer Flüssigkeit zu messen, die durch den Harnkatheter läuft, wobei die handgeführte Volumenmessvorrichtung ein Gehäuse (1) aufweist, das einen ersten Anteil (2) und einen zweiten Anteil (3) aufweist, die schwenkbar miteinander verbunden sind, wobei der erste Anteil (2) und der zweite Anteil (3) jeweils eine Kontaktoberfläche (4, 5) mit einer Einbuchtung (7, 8) umfassen, die durch den Anteil (4, 5) läuft und geformt und dimensioniert ist, um teilweise einen intermittierenden Harnkatheter aufzunehmen, wobei die Einbuchtung in einer geschlossenen Konfiguration der Vorrichtung einen Kanal (6) mit einem Innendurchmesser zwischen 2,7 und 6 mm bildet, wobei das Gehäuse (1) des Weiteren umfasst:
- Flüssigkeitsvolumenmessmittel, die ausgestaltet sind, um das Volumen von Flüssigkeit, die durch den Harnkatheter läuft, über einen Zeitraum zu messen;
- Benutzerinteraktionsmittel (11) zum Aktivieren und/oder Deaktivieren des Flüssigkeitsmessmittels;
- ein drahtloses Übertragungsmittel, das ermöglicht, dass Daten von dem Flüssigkeitsvolumenmessmittel an ein persönliches Endgerät übertragen werden, wie einen Computer, ein Tablet oder ein Smartphone; und
- eine Energiequelle.

2. Handgeführte Volumenmessvorrichtung nach Anspruch 1, wobei das Flüssigkeitsvolumenmessmittel umfasst:
- eine Ultraschallemissionseinheit, die ausgestaltet ist, um mindestens einen Strahl oder Puls von Ultraschall durch einen Teil eines Harnkatheters hindurch zu emittieren,
- eine Ultraschallempfangseinheit, die ausgestaltet ist, um den mindestens einen Strahl oder Puls des Ultraschalls zu empfangen, wenn er durch den Harnkatheter hindurch emittiert worden ist.

3. Handgeführte Volumenmessvorrichtung nach einem der Ansprüche 1 bis 2, wobei die Vorrichtung des Weiteren eine Verarbeitungseinheit umfasst, die ausgestaltet ist, um Daten von dem Flüssigkeitsvolumenmessmittel zu empfangen und die Daten zu verarbeiten, um verarbeitete Daten zu erhalten, die das Volumen von Flüssigkeit, die durch den Harnkatheter läuft, über einen Zeitraum einschließen.

4. Handgeführte Volumenmessvorrichtung nach einem der Ansprüche 1 bis 3, wobei das drahtlose Übertragungsmittel ermöglicht, dass Daten von dem Flüssigkeitsvolumenmessmittel und/oder die verarbeiteten Daten an ein Endgerät übertragen werden, wie einen Computer, ein Tablet oder Smartphone.

5. Handgeführte Volumenmessvorrichtung nach einem der Ansprüche 1 bis 4, wobei das Gehäuse des Weiteren eine Anzeigeeinheit (10) umfasst, die ausgestaltet ist, um verarbeitete Daten von der Verarbeitungseinheit anzuzeigen, die das Volumen einer Flüssigkeit, die durch den Harnkatheter läuft, über einen Zeitraum einschließen.

6. Handgeführte Volumenmessvorrichtung nach einem der Ansprüche 1 bis 5, wobei das Gehäuse (1) eine längliche Form hat und weniger als 15 cm in allen Abmessungen aufweist, wie weniger als 10 cm in allen Abmessungen.

7. Handgeführte Volumenmessvorrichtung nach einem der Ansprüche 1 bis 6, wobei die Vorrichtung eine offene Konfiguration und eine geschlossene Konfiguration aufweist, wobei
- in der offenen Konfiguration der erste Anteil (2) und der zweite Anteil (3) nur durch eine schwenkbares Segment in Kontakt miteinander sind,
- in der geschlossenen Konfiguration die Kontaktoberfläche des ersten Anteils (4) und des zweiten Anteils (5) in Kontakt miteinander sind, so dass die Einbuchtungen (7, 8), die durch den ersten Anteil (2) und den zweiten Anteil (3) laufen, zusammen einen Kanal (6) umschließen, der so eingerichtet ist, dass er um einen Teil einer Außenoberfläche eines Harnkatheters herum gepasst wird.

8. Handgeführte Volumenmessvorrichtung nach einem der Ansprüche 1 bis 7, wobei die Vorrichtung des Weiteren einen Arretiermechanismus (9) umfasst, der so ausgestaltet ist, dass in der geschlossenen Konfiguration der erste Anteil (2) und der zweite Anteil (3) mittels des Arretiermechanismus (9) verriegelt sind.

## Revendications

1. Dispositif portatif de mesure de volume de liquide configuré pour être attaché sur une partie d'un cathéter urinaire intermittent et pour mesurer le volume d'un liquide s'écoulant par le cathéter urinaire, le dispositif portatif de mesure de volume comportant un logement (1) comprenant une première partie (2) et une seconde partie (3) étant reliées de manière pivotante l'une à l'autre, la première partie (2) et la seconde partie (3) comprenant chacune une surface de contact (4, 5) ayant une indentation (7, 8) traversant la partie (4, 5) qui est formée et dimensionnée pour recevoir partiellement un cathéter urinaire intermittent, l'indentation, dans une configuration fermée du dispositif formant un canal (6) ayant un diamètre interne entre 2,7 et 6 mm, le boîtier (1) comprenant en outre
- des moyens de mesure du volume de liquide configurés pour mesurer le volume de liquide s'écoulant à travers le cathéter urinaire sur une période de temps ;
- des moyens d'interaction avec l'utilisateur (11) pour activer et/ou désactiver les moyens de mesure du liquide ;
- un moyen de transmission sans fil, qui permet de transmettre des données depuis les moyens de mesure de volume de liquide jusqu'à un terminal personnel, tel qu'un ordinateur, une tablette ou un téléphone intelligent ; et
- une source d'énergie.

2. Dispositif portatif de mesure de volume selon la revendication 1, dans lequel les moyens de mesure de volume de liquide comprennent
- une unité d'émission d'ultrasons, configurée pour émettre au moins un faisceau ou une impulsion d'ultrasons à travers une partie d'un cathéter urinaire
- une unité de réception d'ultrasons, configurée pour recevoir l'au moins un faisceau ou l'au moins une impulsion d'ultrasons lorsqu'il/elle a été émis(e) à travers le cathéter urinaire.

3. Dispositif portatif de mesure de volume selon l'une quelconque des revendications 1 à 2, dans lequel le dispositif comprend en outre une unité de traitement, configurée pour recevoir des données provenant des moyens de mesure de volume de liquide et traiter les données pour obtenir des données traitées comprenant le volume de liquide s'écoulant par le cathéter urinaire sur une période de temps.

4. Dispositif portatif de mesure du volume selon l'une quelconque des revendications 1 à 3, dans lequel le moyen de transmission sans fil permet de transmettre des données provenant des moyens de mesure de volume de liquide et/ou les données traitées à un terminal, tel qu'un ordinateur, une tablette ou un téléphone intelligent.

5. Dispositif portatif de mesure de volume selon l'une quelconque des revendications 1 à 4, dans lequel le boîtier comprend en outre une unité d'affichage (10), configurée pour afficher des données traitées provenant de l'unité de traitement, y compris le volume d'un liquide s'écoulant par le cathéter urinaire sur une période de temps.

6. Dispositif portatif de mesure de volume selon l'une quelconque des revendications 1 à 5, dans lequel le boîtier (1) a une forme longitudinale et fait moins de 15 cm dans toutes les dimensions, notamment moins de 10 cm dans toutes les dimensions.

7. Dispositif portatif de mesure de volume selon l'une quelconque des revendications 1 à 6, dans lequel le dispositif présente une configuration ouverte et une configuration fermée, dans lequel
- dans la configuration ouverte, la première partie (2) et la seconde partie (3) ne sont en contact l'une avec l'autre que par l'intermédiaire d'une section pivotante,
- dans la configuration fermée, la surface de contact de la première partie (4) et la seconde partie (5) sont en contact l'une avec l'autre de telle sorte que les indentations (7, 8) traversant la première partie (2) et la seconde partie (3) entourent ensemble un canal (6) qui est conçu pour être ajusté autour d'une partie d'une surface externe d'un cathéter urinaire.

8. Dispositif portatif de mesure de volume selon l'une quelconque des revendications 1 à 7, dans lequel le dispositif comprend en outre un mécanisme de verrouillage (9), configuré de telle sorte que, dans la configuration fermée, la première partie (2) et la seconde partie (3) soient interverrouillées au moyen du mécanisme de verrouillage (9).
